(19)

**Europäisches Patentamt**
**European Patent Office**
**Office européen des brevets**

(11) **EP 4 468 304 A1**

(12) **EUROPEAN PATENT APPLICATION**

(43) Date of publication:
**27.11.2024 Bulletin 2024/48**

(21) Application number: **23175844.2**

(22) Date of filing: **26.05.2023**

(51) International Patent Classification (IPC):
**G16H 20/60** (2018.01)

(52) Cooperative Patent Classification (CPC):
**G16H 20/60**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Haleon US Holdings LLC**
**Wilmington, DE 19808 (US)**

(72) Inventors:
• **ZHANG, Wei**
**Pudong, Shanghai 20012 (CN)**

• **CHEN, Wei Jun**
**Shanghai 200124 (CN)**
• **LIU, Ben**
**Shanghai (CN)**
• **ZHANG, Jianfeng**
**Pudong New Distract (CN)**
• **WANG, Jiaxi**
**Shanghai 200124 (CN)**
• **CAO, Tao**
**London SE1 9SH (GB)**
• **LI, Rui**
**Nanjing City (CN)**

(74) Representative: **Haleon Patent Department**
**5 The Heights, Wellington Way**
**Weybridge, Surrey KT13 0NY (GB)**

(54) **METHOD, APPARATUS AND MEDIUM FOR PROVIDING RECOMMENDATION OF PERSONALIZED NUTRITIONAL PRODUCTS**

(57) The present application discloses computer-implemented method, apparatus and medium for providing a recommendation of personalized nutritional products. The method includes: acquiring physiological data information in a plurality of dimensions of a user; generating a plurality of static labels that indicate a health state of the user according to the physiological data information in the plurality of dimensions; acquiring a plurality of behavior data of the user; generating a plurality of dynamic labels that indicate user preferences according to the plurality of behavior data; generating a user portrait based on the static labels and the dynamic labels; generating a plurality of recall lists that indicate correspondence between the user and different nutritional products through a trained recall model based on the plurality of dynamic labels and based on a knowledge graph of nutritional products, and constructing a merged recall list that integrates the plurality of recall lists; constructing a feature vector used for a sorting model by using the merged recall list and the user portrait, and inputting a calculated feature vector into the sorting model; and sorting the merged recall list according to a score calculated by the sorting model for an inputted feature vector, so as to determine the recommendation of personalized nutritional products suitable for the user.

FIG. 2

EP 4 468 304 A1

**Description**

TECHNICAL FIELD

The present disclosure relates to computer-implemented method, apparatus and medium for providing a recommendation of personalized nutritional products, and more particularly to a computer-implemented method for providing a recommendation of personalized nutritional products based on a user portrait and a knowledge graph of nutritional products.

BACKGROUND

At present, with the improvement of living standards, people are more and more concerned about their physical health. For example, people often have regular physical examinations to learn their health state. In addition, with the appearance of wearable devices, people can also use wearable devices to monitor their own sports, sleep and other states. When the body has sub-health symptoms such as poor sleep, dull skin and heavy pressure etc., people may probably see a doctor and take some nutritional products according to the doctor's advice to adjust the body state.

However, in many cases, although the users are sub-healthy, the users feel that going to the hospital is troublesome or it is not serious enough to go to the hospital, so they take some nutritional products according to their own experience, the type, quantity and frequency of nutritional products taken are quite random. This way of taking nutritional products is very inaccurate, and it can't alleviate the symptoms of the users many times. This is because the users determine their physical condition based on vague subjective feelings, and can't accurately determine what nutritional requirements they have. In addition, different nutritional products contain different nutritional elements and aim at different symptoms, ordinary users do not have the corresponding nutritional knowledge, so the users cannot accurately determine which nutritional products should be taken according to their own state data.

In recent years, with the development of genetic testing technique, people can directly know their nutritional requirements through genetic test. However, there is no direct correlation between the nutritional requirements obtained by such tests and the physical condition.

Therefore, how to accurately determine the health state of the users according to the physical state, mental state, genetic test and other data of the users, and provide the users with a recommendation of personalized nutrition products conveniently and accurately has become an urgent problem to be solved.

SUMMARY

Based on this, various embodiments of the present disclosure provide method and apparatus for a recommendation of personalized nutritional products, which method and apparatus are used to provide a recommendation of personalized nutritional products to the users conveniently and accurately.

According to an aspect of the present disclosure, there is provided a computer-implemented method for providing a recommendation of personalized nutritional products, comprising:

acquiring physiological data information in a plurality of dimensions of a user;
generating a plurality of static labels that indicate a health state of the user according to the physiological data information in the plurality of dimensions;
acquiring a plurality of behavior data of the user;
generating a plurality of dynamic labels that indicate user preferences according to the plurality of behavior data;
generating a user portrait based on the static labels and the dynamic labels;
generating a plurality of recall lists that indicate correspondence between the user and different nutritional products through a trained recall model based on the plurality of dynamic labels and based on a knowledge graph of nutritional products, and constructing a merged recall list that integrates the plurality of recall lists;
constructing a feature vector used for a sorting model by using the merged recall list and the user portrait, and inputting a calculated feature vector into the sorting model; and
sorting the merged recall list according to a score calculated by the sorting model for an inputted feature vector, so as to determine the recommendation of personalized nutritional products suitable for the user.

Preferably, the physiological data information in the plurality of dimensions includes one or more of physical examination report data of the user, physiological data of the user received from a wearable device, nutritional requirement data reported by the user himself or herself, and genetic test report data of the user.

Preferably, generating a plurality of static labels that indicate a health state of the user according to the physiological data information in the plurality of dimensions comprises:

generating a first category label with a first weight according to the physical examination report data of the user, the first category label including a plurality of labels that indicate different physiological health states of the user;

generating a second category label with a second weight according to the physiological data of the user received from the wearable device, the second category label including a plurality of labels that indicate different physiological activity states of the user;

generating a third category label with a third weight according to the nutritional requirement data reported by the user himself or herself, the third category label including a plurality of labels that indicate different mental states and nutritional requirements reported by the user himself or herself;

generating a fourth category label with a fourth weight according to the genetic test report data of the user, the fourth category label including a plurality of labels that indicate different long-term nutritional requirements of the user; and

generating the plurality of static labels that indicate the health state of the user according to basic information of the user, the first category label, the second category label, the third category label and the fourth category label, wherein the first weight, the second weight, the third weight and the fourth weight have successively decreasing values.

Preferably, the plurality of behavior data of the user includes a user browsing record, a user searching record, a user question-and-answer record, and a corresponding time record.

Preferably, generating a plurality of dynamic labels that indicate user preferences according to the plurality of behavior data comprises:

generating browsing labels, searching labels and question-and-answer labels according to user browsing records, user searching records and user question-and-answer records of a plurality of users, the labels including three types of entity, keyword and classification;

constructing a plurality of sets with browsing labels, searching labels and question-and-answer labels of the plurality of users in a predetermined time period in units of users;

calculating a score of each user label in the plurality of sets by using a term frequency-inverse document frequency (TF-IDF) algorithm;

generating a final score of each user label according to the score of each user label, a time attenuation of interaction generation, a weight of interaction type and a weight of a label type;

selecting a predetermined number of user labels with top-ranked final scores as the dynamic labels of the user.

Preferably, the recall model comprises:

a singular value decomposition model (SVD)-based SVD recall submodel, which is used to generate an SVD recall list that indicates products that the user is most likely to buy;

an update recall submodel, which is used to generate an update recall list that indicates latest updated products;

a popular recall submodel, which is used to generate a popular recall list of popular products according to a click volume and a time decay of products;

a graph recall submodel, which is used to query products associated with labels from the knowledge graph based on nutritional products according to the plurality of labels of the user, and generate a graph recall list of personalized products; and

a merging submodel, which is used to merge the SVD recall list, the update recall list, the popular recall list and the graph recall list according to a predetermined merging rule so as to generate a merged recall list.

Preferably, constructing a feature vector by using the merged recall list and the user portrait comprises:

performing feature engineering processing on a respective information field in the user portrait; and

performing weight assignment processing according to a weight of a respective feature, to generate a feature vector for the sorting model, wherein the respective weight in the static labels is greater than the respective weight in the dynamic labels.

Preferably, sorting the plurality of recall lists according to a score calculated by the sorting model for an inputted feature vector, so as to determine the recommendation of personalized nutritional products suitable for the user comprises:

calculating, by the sorting model, scores that indicate a degree of the user's interest in products according to the inputted feature vector;

sorting the plurality of recall lists contained in the merged recall listaccording to values of the scores; and

determining the nutritional products in the recall list with the highest score as the recommendation of personalized nutritional products suitable for the user.

Preferably, the personalized nutritional products comprise a plurality of combined product units of different types and different quantities of nutritional products corresponding to the health state of the user.

Preferably, the method further comprises:

determining the health state of the user based on the static labels of the user;
obtaining associated nutritional products based on the health state of the user and the knowledge graph of nutritional products;
determining the recommendation of associated nutritional products suitable for the user, based on the obtained associated nutritional products.

Preferably, the method further comprises:
displaying the recommendation of personalized nutritional products and/or the recommendation of associated nutritional products to the user.

Preferably, the method further comprises:

determining the health state of the user based on the static labels of the user;
displaying a determined health state and a health suggestion corresponding to the health state to the user.

Preferably, the method further comprises:

determining nutritional requirements of the user based on the static labels of the user;
obtaining associated nutritional products based on the nutritional requirements of the user and the knowledge graph of nutritional products;
determining the recommendation of associated nutritional products suitable for the user based on the obtained associated nutritional products.

Preferably, the method further comprises:
displaying the nutritional requirements of the user and/or the recommendation of personalized nutritional products to the user.

According to another aspect of the present disclosure, there is provided an apparatus for providing a recommendation of personalized nutrition products, comprising:

a memory on which a computer program is stored; and
a processor configured to, when executing the computer program, perform:

acquiring physiological data information in a plurality of dimensions of a user;
generating a plurality of static labels that indicate a health state of the user according to the physiological data information in the plurality of dimensions;
acquiring a plurality of behavior data of the user;
generating a plurality of dynamic labels that indicate user preferences according to the plurality of behavior data;
generating a user portrait based on the static labels and the dynamic labels;
generating a plurality of recall lists that indicate correspondence between the user and different nutritional products through a trained recall model based on the plurality of dynamic labels and based on a knowledge graph of nutritional products, and constructing a merged recall listthat integrates the plurality of recall lists;
constructing a feature vector used for a sorting model by using the merged recall listand the user portrait, and inputting a calculated feature vector into the sorting model; and
sorting the merged recall listaccording to a score calculated by the sorting model for an inputted feature vector, so as to determine the recommendation of personalized nutritional products suitable for the user.

According to another aspect of the present disclosure, there is provided a nonvolatile storage medium having stored thereon a computer program which, when executed by a computer, performs the method in the preceding embodiments.

The method and the apparatus for providing the recommendation of personalized nutrition products according to the present disclosure can analyze user data by means of data modeling, machine learning and medical knowledge, thereby providing personalized insight and recommendation for the user. In addition, the method and apparatus for providing the recommendation of personalized nutritional products according to the present disclosure can also obtain recommended nutritional requirements from the genetic test instead of directly investigating the original genetic test data. In addition, the nutritional requirements based on genetic test are also labeled together with other health data to generate a more comprehensive recommendation of personalized nutritional products.

By adopting the method and the apparatus for providing the recommendation of personalized nutritional products disclosed by the present disclosure, the user portrait can be generated according to the static labels that indicate the user's health state and the dynamic labels that indicate the user's behavior, and the recommendation of personalized nutritional products can be conveniently and accurately provided to the user based on the user portrait and the nutritional product knowledge graph.

BRIEF DESCRIPTION OF THE DRAWINGS

The details of one or more implementations are set forth in the appended attachments, drawings and the following description. Other features will be apparent from the specification and drawings and from the claims.

FIG. 1 shows a schematic diagram of a method for providing a recommendation of personalized nutritional products according to a first embodiment of the present disclosure.
FIG. 2 shows a flowchart of a method for providing a recommendation of personalized nutritional products according to the first embodiment of the present disclosure.
FIG. 3 shows a schematic diagram of generating dynamic labels.
FIG. 4 shows a schematic diagram of the knowledge graph of nutritional products.
FIG. 5 shows an example of a sorting model.
FIG. 6 shows a block diagram of an apparatus for providing a recommendation of personalized nutritional product according to a second embodiment of the present disclosure.
FIG. 7 shows a block diagram of a storage medium according to a third embodiment of the present disclosure.

DETAILED DESCRIPTION OF THE EMBODIMENTS

The technical solutions in the embodiments of the present application will be described clearly and completely with reference to the attached drawings. Obviously, these described embodiments are only part of the embodiments of the present application, but not all of the embodiments. Based on the embodiments of the present application, all other embodiments obtained by those of ordinary skilled in the art without paying creative work also belong to the protection scope of the present application.

The terms used in this specification are those general terms that are currently widely used in the art in consideration of the functions related to the present disclosure, however, these terms may change according to the intention of those of ordinary skill in the art, precedents or new techniques in the art. In addition, specific terms can be selected by the applicant, and in this case, their detailed meanings will be described in the detailed description of the present disclosure. Therefore, the terms used in the specification should not be understood as simple names, but rather the general description based on the meanings of the terms and the present disclosure.

Although the present application makes various references to some modules in the system according to the embodiments of the present application, any number of different modules can be used and run on a user terminal and/or a server. The modules are merely illustrative, and different aspects of the system and the method may use different modules.

Flowcharts are used in the present application to explain the operations performed by the system according to the embodiments of the present application. It should be understood that the preceding or following operations are not necessarily performed accurately in order. On the contrary, various steps can be processed in a reverse order or at the same time, as needed. Meanwhile, other operations can be added to these processes, or one or more steps can be removed from these processes.

**First Embodiment**

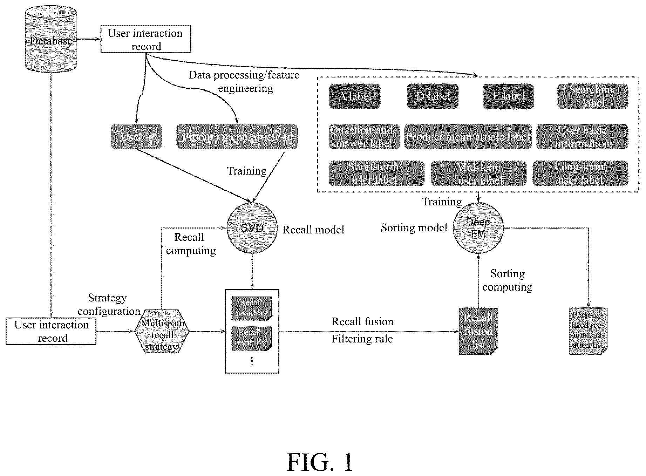

Next, a method of providing a recommendation of personalized nutritional products according to a first embodiment of the present disclosure will be briefly described with reference to FIG. 1.

The method for providing a recommendation of personalized nutritional products according to the first embodiment of the present invention can be realized by a software program such as an APP, for example a mobile App or a WeChat applet.

After the APP is started, the user can register in the APP, and fill in user information including basic information such as gender and age when registering. Thereafter, the user can conduct various evaluations or upload various data of evaluation in the App.

As shown in FIG. 1, various labels (for example, A/D/E labels, etc.) will be generated for the user in each evaluation, and static labels (with a higher weight) of the user will be generated according to these labels.

After the user has accumulated a certain amount of searching records, question-and-answer records, browsing records, dynamic labels (for example, searching labels, question-and-answer labels, short-term user labels, medium-term user labels, long-term user labels, etc.) (the weights are decreasing in turn) of the user will be generated regularly according to the labels of these records.

A user portrait is formed by using the static labels of the user and the dynamic labels of the user.

Item recalling is performed according to the physical state corresponding to the static labels of the user and the behavior records of the user on the whole platform, the items that the user may need, collect, share and browse are initially selected to form a user-item list, and at the same time, multi-channel recalled data, such as popular items and latest items, are generated according to the interaction records of all users.

The user-item list is sorted by using the user portrait and item characteristics as the input of the sorting model, and the sorting result of items that the user may probably want to see/buy is obtained, the sorting result of items is suitable for the user and personalized.

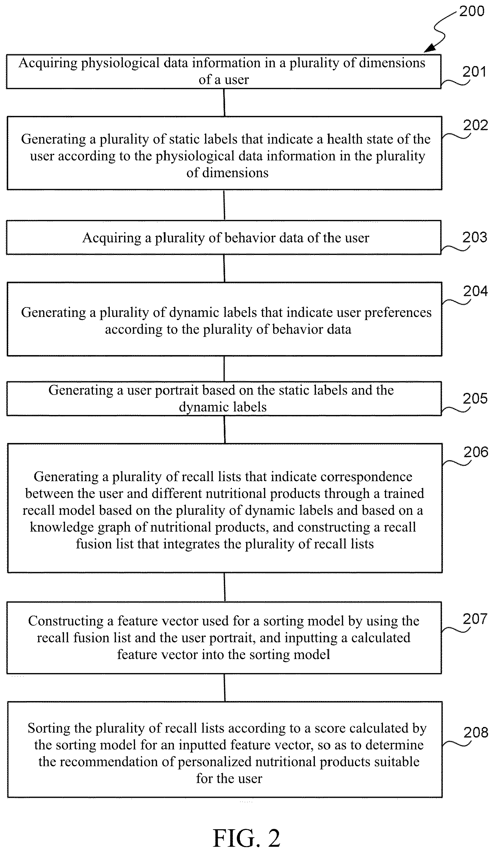

Next, the method of providing a recommendation of personalized nutritional products according to the first embodiment of the present disclosure will be described in detail with reference to FIG. 2.

FIG. 2 is a flowchart showing a method of providing a recommendation of personalized nutritional products in the first embodiment of the present disclosure. As shown in FIG. 2, the method for providing a recommendation of personalized nutrition products includes:

S201: acquiring physiological data information in a plurality of dimensions of a user;

S202, generating a plurality of static labels that indicate a health state of the user according to the physiological data information in the plurality of dimensions;

S203: acquiring a plurality of behavior data of the user;

S204, generating a plurality of dynamic labels that indicate user preferences according to the plurality of behavior data;

S205, generating a user portrait based on the static labels and the dynamic labels;

S206, generating a plurality of recall lists that indicate correspondence between the user and different nutritional products through a trained recall model based on the plurality of dynamic labels and based on a knowledge graph of nutritional products, and constructing a merged recall list that integrates the plurality of recall lists;

S207, constructing a feature vector used for a sorting model by using the merged recall listand the user portrait, and inputting a calculated feature vector into the sorting model;

S208: sorting the merged recall list according to a score calculated by the sorting model for an inputted feature vector, so as to determine the recommendation of personalized nutritional products suitable for the user.

It should be noted that the term "recall model" is used herein to describe a commonly-used model in recommendation applications which generates candidates or retrieves candidates matching certain rules or similarity criteria. Sometimes such a "recall model" is also called a "match model" or "retrieve model".

Specifically, the physiological data information in the plurality of dimensions obtained in step S201 includes one or more of physical examination report data of the user, physiological data of the user received from a wearable device, nutritional requirement data reported by the user himself or herself, and genetic test report data of the user.

For example, the user can fill in the questionnaire provided in the applet and provide nutritional requirements which he or she concerns about. An example of the content of the questionnaire is shown below.

A001 Please select 1 to 4 nutritional requirements that you are most concerned about: [the number of selections is no more than 4 and no less than 1]

A. Skin, hair, and nail
B. Emotional stress
C. Sleep quality
D. Resistance
E. Gastrointestinal health
F. Liver health
G. Sports and body shape management
H. Metabolic activity and fatigue
I. Eyes
J. Exercise and health of bones and joints
K. Cardiovascular health

A002 Age
A003 Gender (male/female)
A004 Weight (kg)
A005 Height (cm)
A006 (If female) Whether are you in the middle of

    A. Pregnancy preparation
    B. Pregnancy
    C. Breastfeeding
    D. Postpartum recovery period (after stopping breastfeeding)
    None. None of the above (single choice)

A007. Taking drugs (Yes/No)
A008. Are there any of the following symptoms in the near future: [multiple choices are allowed, the last one is a single choice]

    A. Night sweats

    B. Hot flashes

    C. Emotional fluctuations

    None. None of the above

A009. Are you taking any of the following drugs: [multiple choices are allowed, the last one is a single choice]

    A. Proton pump inhibitors
    B. Thiazolidinedione hypoglycemic agents
    C. Glucocorticoid drugs
    D. Thyroxine
    E. Antiepileptic drugs
    None. None of the above

A010. What's your recent mental state? [multiple choices are allowed, and the last one is a single choice]

    A. Easy to fatigue
    B. High pressure
    C. Lack of energy
    D. Difficulty in concentration
    E. Feel memory deterioration
    None. None of the above

Only some questionnaire examples are shown above, and other questionnaire questions further include, for example, daily sleep duration, sleep problem, eye problem, hair, skin, nail problem, emotional state, stress degree, various physical discomfort symptoms, diet, living habit, exercise intensity, bone problem, blood sugar, blood, blood lipid disease, recent nutritional deficiency diagnosis and so on.

In addition, the user can upload his or her own medical report in the applet. For example, the physical examination report provides the reference range and detection index values of blood pressure, triglyceride (TG), total cholesterol (TC), fasting blood glucose, alanine aminotransferase (alanine aminotransferase) (ALT), hemoglobin (HGB), carotid color ultrasound and other contents. The following are some examples of submitted reports.

| No. | Project name | Reference range | Unit | High | Low |
|---|---|---|---|---|---|
| B006 | Blood pressure | | | | |

(continued)

| No. | Project name | Reference range | Unit | High | Low |
|---|---|---|---|---|---|
| B007 | Triglyceride (TG) | 0.48-1.88 | Mmol/L | Coronary heart disease; primary hyperlipidemia, atherosclerosis, obesity, diabetes, gout, hypoparathyroidism, nephrotic syndrome etc. | Low β -lipoproteinemia or no low β -lipoproteinemia; severe liver disease, malabsorption, hyperthyroidism, hypoadrenocortical function, etc. |
| B008 | Total cholesterol (TC) | 3.35-6.45 | Mmol/L | There is a possibility of hyperthyroidism, severe liver disease, anemia and malnutrition | It can lead to atherosclerosis and cardiovascular and cerebrovascular diseases; all kinds of hyperlipidemia, cholestatic jaundice, hypothyroidism, lipid nephropathy, diabetes, etc. |

In addition, the user can also configure his or her wearable device (such as the smart watch) to connect with the App to obtain data such as user movement and sleep. In some cases, the smart watch can also be used to obtain data such as blood sugar, blood pressure, heart rate and pressure of the user. The following are some examples of exercise sleep data.

| No. | Decryption | Notes |
|---|---|---|
| C001 | Step number | The mobile phone, the watch, the bracelet and the like all support the daily pedometer data. |
| C002 | Distance | The mobile phone, the watch, the bracelet and the like all support the daily pedometer data. |
| C003 | Calorie | The mobile phone, the watch, the bracelet all support the daily pedometer data. |
| C004 | Heart rate | The watch, the bracelets and the like all support heart rate data. |
| C005 | Sleep state | The watch, the bracelets and the like all support sleep segment detail data. |
| C006 | Blood glucose | blood glucose meter (ecological device) |
| C007 | Blood pressure | blood pressure sphygmomanometer (ecological device) |
| C008 | Pressure | The watch and the high-end bracelet support this data. |

Finally, the user can upload his or her genetic test result to the App. Examples of genetic test indicators are shown below.

| No. | Test item | Detection result |
|---|---|---|
| D001 | Nutritional requirement of Folic acid | High, medium and low |
| D004 | Nutritional requirement of Vitamin D | High, medium and low |
| D005 | Nutritional requirement of Vitamin E | High, medium and low |
| D007 | Nutritional requirement of Vitamin B2 | High, medium and low |
| D009 | Nutritional requirement of Vitamin B6 | High, medium and low |
| D010 | Nutritional requirement of Vitamin B12 | High, medium and low |
| D011 | Nutritional requirement of Calcium | High, medium and low |
| D012 | Nutritional requirement of Ferrum | High, medium and low |
| D013 | Nutritional requirement of Zinc | High, medium and low |
| D022 | Vitamin B1 | High, medium and low |

(continued)

| No. | Test item | Detection result |
|---|---|---|
| D023 | Biotin | High, medium and low |
| D024 | Pantothenic acid | High, medium and low |
| D025 | EPA | High, medium and low |
| D026 | DHA | High, medium and low |
| D030 | Liver health (fatty liver risk) | High, medium and low |
| D031 | Hypertriglyceridemia | High, normal, low |
| D032 | Mixed hyperlipidemia | High, normal, low |
| D033 | Primary hypertension | High, normal, low |
| D034 | Ischemic stroke | High, normal, low |
| D035 | Nonalcoholic fatty liver | High, normal, low |
| D036 | Alcoholic fatty liver | High, normal, low |
| D037 | Type 2 diabetes | High, normal, low |
| D038 | Degenerative arthritis | High, normal, low |
| D039 | Osteoporosis | High, normal, low |
| D040 | Immunity | High, low |
| D041 | Skin | High, low |

In step S202, various static labels of the user can be generated by using these data after the uploading of these data of the user. As shown above, the user fills in the nutrition requirement questionnaire, and the A label is generated according to the questionnaire. The user uploads the physical examination report, and the B label is generated according to the result of the submitted report. The C label is generated according to the data obtained by the wearable device. The user uploads the genetic test report, and the D label is generated according to the result of the genetic test report.

Specifically, a first category label with a first weight is generated according to the physical examination report data of the user, the first category label includes a plurality of labels that indicate different physiological health states of the user. For example, the user uploads a physical examination report and the B label is generated according to the result of the submitted report.

A second category label with a second weight is generated according to the physiological data of the user received from the wearable device, the second category label includes a plurality of labels that indicate different physiological activity states of the user. For example, the C label is generated based on data obtained by the wearable device.

A third category label with a third weight is generated according to the nutritional requirement data reported by the user himself or herself, the third category label including a plurality of labels that indicate different mental states and nutritional requirements reported by the user himself or herself. For example, the user fills in a nutrition requirement questionnaire and the A label is generated according to the questionnaire.

A fourth category label with a fourth weight is generated according to the genetic test report data of the user, the fourth category label includes a plurality of labels that indicate different long-term nutritional requirements of the user. For example, the user uploads a genetic test report and the D label is generated according to the result of the genetic test report.

The plurality of static labels that indicate the health state of the user are generated according to basic information of the user, the first category label, the second category label, the third category label and the fourth category label. For example, according to the algorithm rules of meta-health, an E label is generated based on some or all of the user basic information, the A label, the B label, the C label and the D label.

In this embodiment, these labels have different weights, and the weight values are assigned in the following order: physical examination index > exercise sleep data > questionnaire > gene detection (long-term basic requirement). Therefore, the first weight, the second weight, the third weight and the fourth weight have successively decreasing values.

In an embodiment, each label represents the determination of the user's physical state, which is obtained from the content of the questionnaire and the physical examination report. The label result is 0/1/2, where 0 means that it is impossible to determine that the user matches the label, 1 means that the user is more likely to match the label, and 2 means that the user is confirmed to match the label.

"Questionnaire Answers" in the table below lists all possible answers that can be labeled. For example, for the E006 label, if the user selects the A option of A011 question in the questionnaire, it is considered that insomnia of the user can be determined from the questionnaire. Most labels hit by questionnaire answers will set the label value to 2, and it will be 1 (such as E012) in rare cases.

By default, as long as the determination condition is met, the label value is all 2. If the label value is 1, a description of => 1 will be added at the end. If there are multiple determination criteria that can be labeled (for example, labels can be obtained from the questionnaire answers and the physical examination report concurrently), the label result takes the maximum value.

Some examples of the E label are shown below.

| No. | Label name | Questionnaire answer | Physical examination report or basic information | Sports label |
|---|---|---|---|---|
| E001 | Anaemia | A024-A | B013-low B014-low | |
| E003 | Dull skin | A014-ABCDE Female at 40+ | | C005(<6h) or Direct conclusion of bracelet (1) |
| E004 | Menopause night sweat hot flash | A008-AB Female of [45, 60] => 1 | | |
| E005 | Emotional stress | A015-ABC A016-AB Female of [45, 60] => 1 | | C008() (2) |
| E006 | Insomnia | A011-A A012-ABC | Female of [45, 60] && B012-high => 1 | C005(<6h or Direct conclusion of bracelet) (2) |
| E007 | Bad joints | A023-ABD Female at 40+ && bmi>24 | | |
| E008 | Fatigue-prone | A010-A | B014-low && B012-high => 1 | |
| E009 | High mental stress from work | A010-BC | | C008 (2) |
| E010 | Easy to catch a cold | A017-AD A027-A | | C008(High) (1) |
| E011 | Lactose intolerance | A020-D | | |
| E012 | Insufficient intake of fruits and vegetables | A020-B => 1 | | |
| E013 | Suckling period | A006-C | | |
| E014 | Shaping and reducing fat | A020-CE bmi>24 => 1 | | |
| E015 | Bone mineral density improvement | A023-CEF | | |
| E017 | Sports enthusiast | A022-ABC | | C001 (2) C003 (2) C012(Analysis result) (2) |
| E021 | Prostate problem | Male at 50+ => 1 | B016-true | |

(continued)

| No. | Label name | Questionnaire answer | Physical examination report or basic information | Sports label |
|-----|-----------|---------------------|------------------------------------------------|-------------|
| E022 | Liver health | A021-A | B012-high B019-AB | |
| E023 | Muscle weakness | A023-G Male and Female at 60+ => 1 | | |
| E024 | Poor memory | A010-DE Male and Female 60+ => 1 | | |

Some or all of the basic user information, the A label, the B label, the C label, the D label and the E label form the static labels of user. The following is an example of the static labels.

```
{
    "userId": "haleon_user_9", # user id
    "sex": "male", # gender
    "birthday": "1990-01-17", # birthday
    "height": NumberInt("160"), # height
    "weight": NumberInt("100"), # weight
    "instances": [ # A, D, E label
        {
            "instanceName": "emotion, stress",
            "sourceName": "survey",
            "objectName": "health requirement" },
        {
            "instanceName": "constipation, diarrhea",
            "sourceName": "huawei",
            "objectName": "health performance" },
        {
            "instanceName": "dull skin",
            "sourceName": "huawei",
            "objectName": "health performance" } ], }
```

In step S203, a plurality of behavior data of the user can be acquired. The plurality of behavior data of the user includes a user browsing record, a user searching record, a user question-and-answer record, and a corresponding time record. For example, after the user has used the program for a period of time, some user interaction records will be generated. The interaction records include "browsing products", "searching records" and "viewing question-and-answer pairs". These interaction records are the behavior data of the user.

Then, in step S204, generating a plurality of dynamic labels that indicate user preferences according to the plurality of behavior data comprises:

generating browsing labels, searching labels and question-and-answer labels according to user browsing records, user searching records and user question-and-answer records of a plurality of users, the labels including three types of entity, keyword and classification;

constructing a plurality of sets with browsing labels, searching labels and question-and-answer labels of the plurality of users in a predetermined time period in units of users;

calculating a score of each user label in the plurality of sets by using a term frequency-inverse document frequency (TF-IDF) algorithm;

generating a final score of each user label according to the score of each user label, a time attenuation of interaction generation, a weight of interaction type and a weight of a label type;

selecting a predetermined number of user labels with top-ranked final scores as the dynamic labels of the user.

Specifically, for example, the embodiment of the present application will generate dynamic labels such as the "searching label", the "question-and-answer label", the "short-term user label (7 days)", the "medium-term user label (30 days)" and the "long-term user label (365 days)" according to the user interaction records.

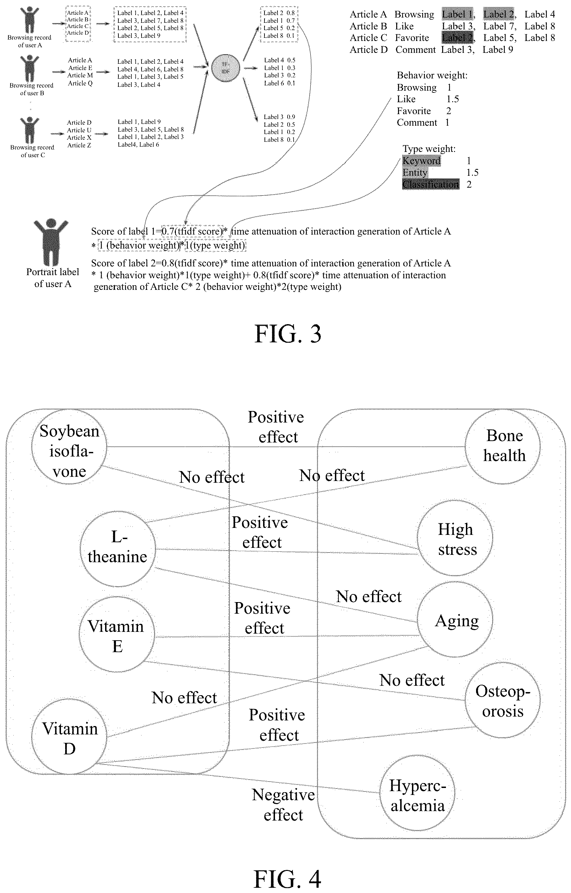

FIG. 3 shows the generation process of dynamic labels. As shown in FIG. 3, the dynamic label generation steps are as follows:

a. Collections are set up respectively for items/product labels recorded by the user searching/questioning-and-answering pair/browsing (7 days)/browsing (30 days)/browsing (365 days) on the whole platform within a predetermined time window, in units of users, for example

> User A: [Label 1, Label 2, Label 5, Label 9]
> User B: [label 3, label 4, label 7]
> User C: [Label 2, Label 6, Label 8, Label 10]

In this embodiment, labels are divided into three types, such as "entity", "keyword" and "classification".

b. The term frequency-inverse document frequency (TF-IDF) algorithm is used to calculate the score of each user label.

c. The tf-idf score, the time decay, the interaction type (view, like, favorite) and the label type (entity, keyword, classification) are combined to calculate the score of each user label, and the labels with the top 10 or top 5 scores are taken as the dynamic labels of the users.

In this embodiment, for example, user A's Label 1 score = tf-idf score * Time attenuation of interaction generation * Interaction type weight * Label type weight.

Next, an example of the dynamic labels of the user is shown.

```
{
    "topNLabels": [
        {
            "label": "insomnia",
            "score": 0.8 },
        {
            "label": "fatigue",
            "score": 0.6 } ],
    "midTopNLabels": [
        {
            "label": "skin",
            "score": 0.6 },
        {
            "label": "constipation",
            "score": 0.2 } ],
        "longTopNLabels": [
        {
            "label": "health",
            "score": 0.5 },
        {
            "label": "movement",
            "score": 0.4 } ],
        "searchTopNLabels": [
        {
            "label": "sleep quality",
            "score": 0.5 },
        {
            "label": "emotional stress",
            "score": 0.2 } ],
        "qaTopNLabels": [
        {
            "label": "metabolic vitality and fatigue",
            "score": 0.3 },
        {
            "label": "resistance",
            "score": 0.1 } ] }
```

In step S205, a user portrait may be generated based on the static labels and the dynamic labels that are generated. The user portrait is a style composed of static label+dynamic label+user information in the form of labels.

Then, in step S206, a plurality of recall lists that indicate correspondence between the user and different nutritional products are generated through a trained recall model based on the plurality of dynamic labels and based on a knowledge graph of nutritional products, and a merged recall list that integrates the plurality of recall lists is constructed.

Specifically, the recall model may include a plurality of recall submodels for recalling various nutritional products associated with the user. For example, the recall model may include a singular value decomposition model (SVD)-based SVD recall submodel, an update recall submodel, a popular recall submodel and a graph recall submodel.

The SVD recall submodel is used to generate an SVD recall list indicating products that the user is most likely to buy. The scoring matrix used in the SVD model is composed of users and items. The following table is an example.

|  | User 1 | User 2 | User 3 | User 4 | User 5 |
|---|---|---|---|---|---|
| Item A |  | 4 | 3 |  | 2 |
| Item B | 5 |  | 1 |  |  |
| Item C |  | 2 |  |  | 4 |

The given scoring matrix S is decomposed into the product of three matrices, where U and V are called left and right singular vectors, which can be understood as the user matrix and the item factor matrix in this embodiment. The value on the diagonal is called the singular value; S is a matrix of n*m, U is a matrix of n*n, Q is a matrix of n*m, and V is a matrix of m*m. In the following formula, k singular values can be used to approximately replace the R matrix, because the sum of the first 1% singular values accounts for more than 99% of the sum of all singular values, because all other singular values are basically 0 except the middle singular value. The formula is as follows:

$$S_{n*m} = U_{n*n} * Q_{n*m} * V_{m*n} \qquad S_{n*m} \approx U_{n*k} * Q_{k*k} * V_{k*n} \quad \text{Formula (1)}$$

Then the U and V matrices need to be transformed to get the user factor matrix C and the item factor matrix P, the square root of the singular matrix Q is taken and to be multiplied to the U and V matrices respectively, just as that the singular matrix Q is transformed by a square root and then a product. The user factor matrix C and the item factor matrix P are obtained as follows:

$$C_{n*k} = U_{n*k} * (Q_{k*k})^{1/2} \qquad P_{m*k} = V_{m*k}^{T} * (Q_{k*k})^{1/2} \quad \text{Formula (2)}$$

Finally, the score prediction of the user t for item i is that the t row of the user factor matrix is multiplied by the i column of the item factor matrix (that is, transposition of the i row in the item factor matrix), as shown below:

$$R_{t,i} = C_{t}Q_{i}^{T}; (1 \leq t \leq n, 1 \leq i \leq n) \quad \text{Formula (3)}$$

The update recall submodel is used to generate an update recall list that indicates latest updated products.

The popular recall sub-model is used to generate a popular recall list of popular products according to a click volume and a time decay of products.

For example, the update recall submodel selects the top n ones as the popular recall objects according to the product/article release time sorted in a reverse order. The popular recall formula uses, for example, the following formula:

$$heat = \begin{cases} \sum_{i}^{n} max(e^{-0.099t_i}, 0.01)r_{count} , & 0 \leq t \leq 3 \\ \sum_{i}^{n} max(e^{-0.099t_i}, 0.01)log(r_{count}) , & 3 < t \leq 7 \\ \sum_{i}^{n} (31 - t_i)/30000 + 0.00001r_{count} , & 7 < t \leq 30 \\ \sum_{i}^{n} (366 - t_i)/365000 , & 30 < t \leq 365 \\ n/365000 , & 365 < t \end{cases}$$

As shown in the above formula, *heat* is the popularity of the article/product, *n* is the total number of interactions, $t_i$ is the time when the *i*-th interaction occurs, and $r_{count}$ is the amount of user interactions (if there is only browsing, then $r_{count}$ is 1, if there is browsing and collecting as favorite, then $r_{count}$ is 2).

The graph recall submodel is used to query products associated with labels from the knowledge graph based on nutritional products according to the plurality of labels of the user, and generate a graph recall list of personalized products.

FIG. 4 shows a knowledge graph based on nutritional products. The knowledge graph based on nutritional products adopts the triplet in way of < entity, relationship, entity >.

The nutritional knowledge graph includes the correlation between a plurality of nutritional elements and at least one health state, and one type of correlation in the nutritional knowledge graph represents the influence result of a nutritional element on a health state. The effect of a nutrient element on a nutritional index can include positive effect, negative effect or no effect, where the positive effect means that the nutrient element contributes to the health state and has a gain effect on the health state; the negative influence means that the nutrient element is harmful to the health state and has a negative effect on the health state; the no effect means that the nutrient element has no effect on the health state.

As shown in FIG. 4, for example, soybean isoflavone non-glycoside contributes to bone health, L-theanine helps to relieve the tension caused by stress, vitamin E acts as an antioxidant to protect cells from free radicals, vitamin D has an adverse effect on hypercalcemia, and so on.

The graph recall sub-model can retrieve the corresponding plurality of nutrient elements from the graph according to the user's plurality of labels (such as E labels). Then, a plurality of nutritional products corresponding to the plurality of nutritional elements are searched in the database. Finally, a graph recall list of personalized users-nutritional products specific to each user is formed according to the retrieval result.

The recall model may further include a merging submodel, which is used to merge the SVD recall list, the update recall list, the popular recall list and the graph recall list according to a predetermined merging rule so as to generate a merged recall list.

Examples of recall merging rules are, for example, as shown in the following table, assuming there are three recall strategies and five products, product A is recalled by all recall strategies 1, 2 and 3, product C is also recalled by all the three recall strategies, with products A and C scoring 3, product B is recalled only by recall strategies 2 and 3, with product B scoring 2, then only products A and B and C will be recalled if the recall quantity is 3.

|  | Recall strategy 1 | Recall strategy 2 | Recall strategy 3 | Total score |
|---|---|---|---|---|
| **A** | √ | √ | √ | 3 |
| **B** |  | √ | √ | 2 |
| **C** | √ | √ | √ | 3 |
| **D** | √ |  |  | 1 |
| **E** |  |  | √ | 1 |

In step S207, a feature vector used for a sorting model is constructed by using the merged recall list and the user portrait, and a calculated feature vector is inputted into the sorting model.

Specifically, constructing a feature vector by using the merged recall list and the user portrait comprises: performing feature engineering processing on a respective information field in the user portrait. Because the input data of the sorting model has a specific format, it is necessary to perform feature engineering processing on each information field in the user portrait in order to obtain formatted information.

An example of the user portrait is shown below.

|  | User A |
|---|---|
| Age | 40 |
| Height | 165 |
| Weight | 104 |
| Gender | Female |
| A label | Emotion, stress, sleep quality, vision and eye health, exercise and bone and joint health |
| D label | Calcium nutrition requirement, DHA requirement, skin comprehensive ability |
| E label | Difficult to fall asleep, easy to get tired, stressful at work, and sports enthusiast |
| Searching label | Sleep quality, emotional stress |
| Question-and-answer label | Metabolic vitality and fatigue, resistance |
| Short-term user label | Insomnia, fatigue |
| Mid-term user label | Skin, constipation |
| Long-term user label | Health, exercise |
| Product entity label | Protein |
| Product keyword label | Cortisol, aldosterone, health care expert, free form, bile acid |
| Product category label | Cardiovascular health |

The model input after feature processing.

| | User A |
|---|---|
| Age | 0.4 (normalized) |
| Height | 0.78 (normalized) |
| Weight | 0.65 (normalized) |
| Gender | 001 (001: female, 010: male, 100; other) |
| A label | 01001000100 (there are 11 dimensions in total, and a value of 1 in the dimension means that there is this label). |
| D label | 00001100000 ... (there are 23 dimensions in total, and a value of 1 in the dimension means that there is this label) |
| E label | 10100000100 ... (there are 24 dimensions in total, and a value of 1 in the dimension means that there is this label ) |
| Searching label | [0.23, 0.31, 0.22, 0.12, 0.64] (embedding for each word, summing and averaging) |
| Question-and-answer label | [0.56, 0.83, 0.12, 0.29, 0.23] (embedding for each word, summing and averaging) |
| Short-term user label | [0.43, 0.51, 0.12, 0.42, 0.18] (embedding for each word, summing and averaging) |
| Mid-term user label | [0.16, 0.21, 0.08, 0.25, 0.24] (embedding for each word, summing and averaging) |
| Long-term user label | [0.14, 0.2,0.63,0.13,0.24] (embedding for each word, summing and averaging) |
| Product entity label | [0.43, 0.61, 0.25, 0.02, 0.06] (embedding for each word, summing and averaging) |
| Product keyword label | [0.13, 0.32, 0.24, 0.12, 0.14] (embedding for each word, summing and averaging) |
| Product category label | [0.21, 0.12, 0.33, 0.34, 0.04] (embedding for each word, summing and averaging) |

Then, weight assignment processing is performed according to a weight of a respective feature, to generate a feature vector for the sorting model, wherein the respective weight in the static labels is greater than the respective weight in the dynamic labels.

Model input after weight assignment

| | User A |
|---|---|
| Age | 0.4 (normalized) |
| Height | 0.78 (normalized) |
| Weight | 0.65 (normalized) |
| Gender | 001 (001: female, 010: male, 100: other) |
| A label | 0 0.8 0 0 0.8 0 0 0 0.8 0 0 (weight 0.8) |
| D label | 0 0 0 0 0.6 0.6 0 0 0 0 0... (weight 0.6) |
| E label | 0.5 0 0.5 0 0 0 0 0 0.5 0 0... (weight 0.5) |
| Searching label | [0.23, 0.31, 0.22, 0.12, 0.64]*0.5 (weight 0.5) |
| Question-and-answer label | [0.56, 0.83, 0.12, 0.29, 0.23]*0.5 (weight 0.5) |
| Short-term user label | [0.43, 0.51, 0.12, 0.42, 0.18]*0.5 (weight 0.5) |
| Mid-term user label | [0.16, 0.21, 0.08, 0.25, 0.24]*0.3 (weight 0.3) |
| Long-term user label | [0.14, 0.2, 0.63, 0.13, 0.24]*0.2 (weight 0.2) |
| Product entity label | [0.43, 0.61, 0.25, 0.02, 0.06] |
| Product keyword label | [0.13, 0.32, 0.24, 0.12, 0.14] |
| Product category label | [0.21, 0.12, 0.33, 0.34, 0.04] |

In step S208, the plurality of recall lists contained in the merged recall listare sorted according to a score calculated by the sorting model for an inputted feature vector, so as to determine the recommendation of personalized nutritional products suitable for the user.

Specifically, the sorting model calculates scores that indicate a degree of the user's interest in products according to the inputted feature vector; and sorts the plurality of recall lists according to values of the scores.

Finally, the sorting model determines the nutritional products in the recall list with the highest score as the recommendation of personalized nutritional products suitable for the user. In other words, the sorting model scores the user's "level of interest" in this product according to the input, and the recall list with a higher score will be presented to the user first.

The sorting model is based on the DeepFM model, for example. The DeepFM model combines the advantages of breadth and depth models, and jointly trains the FM model and the DNN model to learn both the low-order feature combination and the high-order feature combination. In addition, the Deep component and the FM component of the DeepFM model share data input from the Embedding layer. DeepFM model is the commonly-used model in Neural network. The detail information can be referenced to "DeepFM: A Factorization-Machine based Neural Network for CTR Prediction, IJCAI 2017", the whole content of which is incorporated herein by reference.

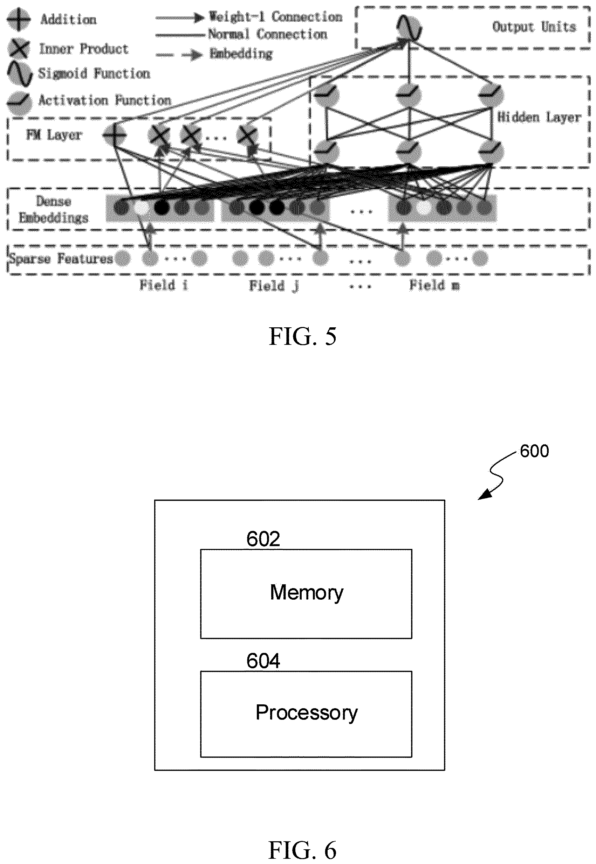

FIG. 5 shows a system block diagram of DeepFM. DeepFM consists of two parts, the left FM part and the right DNN part. These two parts share the same input. For a given feature i, wi is used to indicate the importance of the first-order feature, and the latent vector Vi of a feature ii is used to indicate the interaction with other features. In the FM part, Vi is used to characterize the second-order feature, while in the neural network part, it is used to construct the higher-order feature. For the current model, all parameters participate in the training together. The prediction result of DeepFM can be written as $y \in (0,1)$, which is the predicted CTR, the result derived for the FM part and the result for the DNN part.

Specifically, DeepFM includes two parts, the left FM part and the right DNN part, and its prediction formula can be written as

$$\hat{y} = y_{FM} + y_{DNN}$$

where YFj'.f is:

$$y_{FM} = <w, x> + \sum_{j_1=1}^{d} \sum_{j_2=j_1+1}^{d} <v_i, v_j> x_{j_1} \cdot x_{j_2}$$

where $w \in R^d$, $x$ is the inputted feature, $<w,x>$ is the adding unit to calculate the first-order feature, and for a given feature $i$, $v_i$ is used to represent the interaction with other features and the second-order feature;

where $y_{DNN}$ is:

$$y_{DNN} = \sigma(W^{|H|+1} \cdot a^H + b^{|H|+1})$$

where $|H|$ is the number of hidden layers of the DNN neural network, $W$ is the weight of the DNN neural network, $b$ s the bias term of the DNN neural network, and $a$ is the inputted feature.

The sorting model calculates scores that indicate a degree of association between products and users according to the inputted feature vector, and sorts the plurality of recall lists according to values of the scores. Finally, the sorting model can determine the nutritional products in the recall list with the highest score as the recommendation of personalized nutritional products suitable for the user.

In a preferred embodiment, the personalized nutritional products recommended to the user according to the user portrait include a plurality of combined product units of different types and different quantities of nutritional products corresponding to the health state of the user. In this way, the user can directly learn the personalized products they need without going to the hospital to see a doctor.

The following shows an example of combined products for different user portraits.

| No. | Portrait of crowds | Product combination | E label (symptom label) | D label (gene label) | A001 (requirement label) |
|---|---|---|---|---|---|
| FB013 | Health basic style (no three highs, namely: high blood pressure, high cholesterol, high blood sugar) | CALTRATE® calcium vitamin D soft capsule (EC version)+ TREERLY brand multivitamin tablet 550mg*60 tablets/bottle+protein powder 10G × 24 bags+CENTRUM Tiancan® melatonin vitamin B6 soft capsule 90 tablets. | Dull skin (need for beauty care) (E003) Emotional stress (E005) Insomnia (poor sleep) (E006) Easily tired/weak/-tired (E008) High stress at work (nervousness) (E009) Insufficient intake of fruits and vegetables (E012) Bone mineral density improvement (E015) | Folic acid nutritional requirement(D001) Vitamin D nutritional requirement (D004) Vitamin B2 nutritional requirement (D007) Vitamin B6 nutritional requirement (D009) Calcium Nutrition Requirement (D011) Vitamin B 1 (D022) Osteoporosis (D039) | A001 a) Skin, hair and nail A001 b) Emotion and stress A001 c) Sleep quality A001 h) Metabolic activity and fatigue A001 j) Exercise and health of bones and joints |
| FB014 | Basic model+blood lipid+vegetarian diet | Calch calcium vitamin D soft capsule (EC version)+Qianlin brand B vitamins 550mg*60 tablets/bottle+10Gx24 bags of protein powder+Qianlin brand fish oil soft capsule 1g*200 tablets/bottle. | Stress at work (nervousness) (E009) Easily tired/weak/tired (E008) Bone mineral density improved (E015) Atherosclerosis (E027) Hyperlipidemia = (E028) Vegetarian (E031) | Folic acid nutritional requirement (D001) Vitamin D nutritional requirement (D004) Vitamin B2 nutritional requirement (D007) Vitamin B6 nutritional requirement (D009) Calcium Nutrition Requirement (D011) Vitamin B1(D022) | A001 d) resistance A001 j) Exercise and health of bones and joints A001 h) Metabolic activity and fatigue |

In a preferred embodiment, it is also possible to determine the health state of the user based on the static labels of the user, to obtain associated nutritional products based on the health state of the user and the knowledge graph of nutritional products, and to determine the recommendation of associated nutritional products suitable for the user, based on the obtained associated nutritional products.

After obtaining the recommendation of personalized nutritional products for the user, the recommendation of personalized nutritional products and/or the recommendation of associated nutritional products can also be displayed to the user through the App.

In a preferred embodiment, it is also possible to determine the health state of the user based on the static labels of the user, and to display a determined health state and a health suggestion corresponding to the health state to the user. For example, the determined health state and health suggestion corresponding to the health state can be displayed to the user through the APP.

In a preferred embodiment, it is also possible to determine nutritional requirements of the user based on the static labels of the user, to obtain associated nutritional products based on the nutritional requirements of the user and the knowledge graph of nutritional products, and to determine the recommendation of associated nutritional products suitable for the user based on the obtained associated nutritional products.

For example, the nutritional requirement of the user and/or the recommendation of the personalized nutritional product can be displayed to the user through the APP.

In a preferred embodiment, for example, the above method of determining nutritional requirements of the user, determining the recommendation of associated nutritional products suitable for the user and/or determining the health state of the user and the like can be implemented on a remote server.

Then, the remote server can transmit the determined nutritional requirements of the user, determined the recommendation of associated nutritional products suitable for the user and/or determined the health state of the user and the like to an electronic device of the user, so as to display on the electronic device of the user.

The method for providing the recommendation of personalized nutrition products according to the present disclosure can analyze user data by means of data modeling, machine learning and medical knowledge, thereby providing personalized insight and recommendation for the user. In addition, the method for providing the recommendation of personalized nutritional products according to the present disclosure can also obtain recommended nutritional requirements from the genetic test instead of directly investigating the original genetic test data. In addition, the nutritional requirements based on genetic test are also labeled together with other health data to generate a more comprehensive recommendation of personalized nutritional products.

Therefore, by adopting the method for providing the recommendation of personalized nutritional products of the first embodiment of the present disclosure, the user portrait can be generated according to the static labels that indicate the user's health state and the dynamic labels that indicate the user's behavior, and the recommendation of personalized nutritional products can be conveniently and accurately provided to the user based on the user portrait and the nutritional product knowledge graph.

< Second Embodiment >

Next, an apparatus for providing a recommendation of personalized nutritional products according to a second embodiment of the present disclosure will be described with reference to FIG. 6.

As shown in FIG. 6, an apparatus 600 for providing a recommendation of personalized nutritional products according to the second embodiment includes a memory 601 and a processor 602.

The memory 601 stores a computer program.

The processor 602 can execute the computer program on the memory 601. When the processor executes the program, it can execute:

acquiring physiological data information in a plurality of dimensions of a user;
generating a plurality of static labels that indicate a health state of the user according to the physiological data information in the plurality of dimensions;
acquiring a plurality of behavior data of the user;
generating a plurality of dynamic labels that indicate user preferences according to the plurality of behavior data;
generating a user portrait based on the static labels and the dynamic labels;
generating a plurality of recall lists that indicate correspondence between the user and different nutritional products through a trained recall model based on the plurality of dynamic labels and based on a knowledge graph of nutritional products, and constructing a merged recall listthat integrates the plurality of recall lists;
constructing a feature vector used for a sorting model by using the merged recall listand the user portrait, and inputting a calculated feature vector into the sorting model; and
sorting the merged recall listaccording to a score calculated by the sorting model for an inputted feature vector, so as to determine the recommendation of personalized nutritional products suitable for the user.

When such apparatus 600 executes a program, the method described in the first embodiment above is executed. A detailed description thereof is omitted here.

The apparatus 600 may further include a display, which can display to the user the recommendation of personalized nutritional products suitable for the user.

The apparatus 600 is, for example, an electronic device with computing power such as a mobile terminal, a desktop computer, a notebook computer, a server, and a portable computing device etc.

The apparatus 600 is, for example, a system including multiple separate components. For example, one processing component can be disposed on or implemented as a remote server, and a display and/or another processing component can be disposed on a local mobile terminal. The remote server can transmit the processed information to the local mobile terminal to display.

Therefore, with the apparatus for providing a recommendation of personalized nutritional products in the second embodiment of the present disclosure, the user portrait can be generated according to the static labels that indicate the health state of the user and the dynamic labels that indicate the behavior of the user, and the recommendation of personalized nutritional products can be conveniently and accurately provided to the user based on the user portrait and the nutritional product knowledge graph.

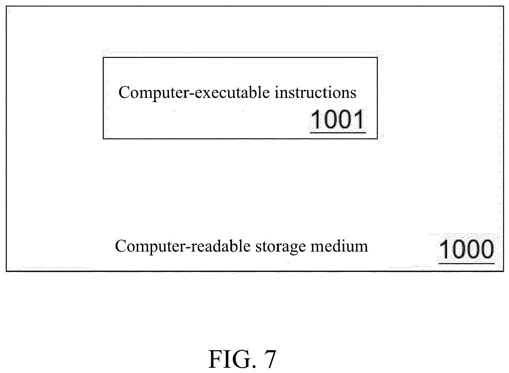

Based on the above embodiments, an embodiment of the present disclosure further provides a computer-readable storage medium. FIG. 7 shows a schematic diagram 1000 of a storage medium according to an embodiment of the present disclosure. As shown in FIG. 7, the computer-readable storage medium 1000 has stored thereon computer-executable instructions 1001. When the computer-executable instructions 1001 are executed by a processor, the method of providing a recommendation of personalized nutritional products in the above embodiment can be realized.

The computer-readable storage medium includes, but is not limited to, for example, volatile memory and/or nonvolatile memory. The volatile memory may include, for example, a random access memory (RAM) and/or a cache, and the like. The nonvolatile memory may include, for example, a read-only memory (ROM), a hard disk, a flash memory, and the like.

An embodiment of the present disclosure further provides a computer program product or computer program including computer instructions stored in a computer-readable storage medium. The processor of the computer device reads the computer instructions from the computer-readable storage medium, and the processor executes the computer instructions, so that the computer device realizes the apparatus for providing a recommendation of personalized nutrition products in the above embodiment.

Those skilled in the art can understand that there may be many variations and improvements to the content disclosed in the present disclosure. For example, various devices or components described above can be realized by hardware, software, firmware, or a combination of some or all of the three.

Furthermore, although the present disclosure makes various references to some units in the system according to the embodiments of the present disclosure, any number of different units can be used and run on a client and/or a server. The units are merely illustrative, and different units may be used for different aspects of the system and method.

One of ordinary skill in the art can understand that all or part of the steps in the above method can be completed by instructing related hardware through a program, the program can be stored in a computer-readable storage medium, such as a read-only memory, a magnetic disk or an optical disk, and the like. Optionally, all or part of the steps of the above embodiments can also be realized by using one or more integrated circuits. Accordingly, the respective module/unit in the above embodiments can be implemented in the form of hardware or software functional modules. The present disclosure is not limited to any particular form of combination of hardware and software.

In summary, it should be understood that, although specific embodiments of the disclosed technique have been described herein for the purpose of explanation, various modifications can be made without departing from the scope of the present disclosure. Therefore, except for the appended claims, the technique of the present disclosure is not limited.

The implementation of the subject matter and functional operations described in this patent document can be implemented in various systems, digital electronic circuits, or in computer software, firmware or hardware (including the structures disclosed in the specification and their structural equivalents), or in the combination of one or more of them. Implementations of the subject matter described in this specification may be implemented as one or more computer program products, i.e., one or more modules of computer program instructions encoded on tangible and non-transitory computer-readable media, for execution by or control of the operation of a data processing device. The computer-readable medium may be a machine-readable storage device, a machine-readable storage substrate, a memory device, a composition of a substance influencing a machine-readable propagation signal, or a combination of one or more of them. The term "data processing unit" or "data processing device" covers all devices, equipment and machines for processing data, including, for example, a programmable processor, a calculator, or a plurality of processors or computers. In addition to hardware, the device may also include codes that create an execution environment for the mentioned computer program, for example, codes that constitute a processor firmware, a protocol stack, a database management system, an operating system, or a combination of one or more of them.

A computer program (also known as a program, software, software disclosure, script or code) can be written in any form of programming language, including assembly or interpretation language, and it can be deployed in any form, including as a stand-alone program or module, a component, a subroutine or other unit suitable for use in a computing environment. A computer program does not have to correspond to a file in a file system. A program may be stored in part of a file with other programs or data (for example, one or more scripts stored in a file in a markup language), in a single file dedicated to the mentioned program, or in a plurality of coordinated files (for example, a file storing one or more modules, subroutines, or partial codes). A computer program can be deployed to be executed on one computer or a plurality of computers located at one site or distributed across multiple sites and interconnected by a communication network.

The processors and logic flows described in this specification can be executed by one or more programmable processors executing one or more computer programs to perform functions by manipulating input data and generating outputs. The processor and the logic flows can also be executed by a dedicated logic circuit, and the apparatus can also be implemented as a dedicated logic circuit, such as an FPGA (Field Programmable Gate Array) or an ASIC (Application Specific Integrated Circuit).

Processors suitable for executing computer programs include, for example, general-purpose and special-purpose microprocessors, and any one or more processors of any kind of digital computers. Typically, a processor will receive instructions or data from a read-only memory or a random access memory or both. The essential elements of a computer are a processor for executing instructions and one or more memory devices for storing instructions and data. Generally, the computer will also include one or more mass storage devices for storing data, such as magnetic disks, magneto-optical disks or optical disks, or be operatively coupled to receive data from or transmit data to one or more mass storage devices for storing data. However, computers do not necessarily have these devices. Computer-readable media suitable for storing computer program instructions and data include all forms of non-volatile memory, media and memory devices, including, for example, semiconductor memory devices (e.g., EPROM, EEPROM and flash memory devices).

The processor and memory can be supplemented by or incorporated into dedicated logic circuits.

The specification and drawings are to be regarded as exemplary only, wherein being exemplary refers to an example. As used herein, the singular forms "a", "an" and "the" are also intended to include the plural forms unless the context clearly indicates otherwise. Furthermore, unless the context clearly indicates otherwise, the use of "or" is also intended to include "and/or".

Although this document contains many details, these should not be construed as limitations on the scope of any invention or claims, but rather as descriptions of features of specific embodiments specific to specific inventions. Some features described in this patent document in the context of a single embodiment can be implemented in a combined manner in a single embodiment. On the contrary, various features described in the context of a single embodiment can also be implemented in multiple embodiments alone or in any suitable sub-combination. Furthermore, although some features may be described above as functioning in certain combinations and even claimed as such at first, one or more features from the claimed combinations may be removed from the combinations in some cases, and the claimed combinations may involve subcombinations or variations of subcombinations.

Similarly, although the operations are described in a particular order in the drawings, this should not be understood as the need to perform such operations in the particular order or sequence shown, or the need to perform all the operations shown to achieve the desired result. Furthermore, the division of various system components in the embodiments described in this patent document should not be understood as requiring such division in all embodiments.

Only some implementations and examples have been described, and other implementations, enhancements and changes can be made based on what is described and illustrated in this patent document.

## Claims

1. A computer-implemented method for providing a recommendation of personalized nutritional products, comprising:

   acquiring physiological data information in a plurality of dimensions of a user;
   generating a plurality of static labels that indicate a health state of the user according to the physiological data information in the plurality of dimensions;
   acquiring a plurality of behavior data of the user;
   generating a plurality of dynamic labels that indicate user preferences according to the plurality of behavior data;
   generating a user portrait based on the static labels and the dynamic labels;
   generating a plurality of recall lists that indicate correspondence between the user and different nutritional products through a trained recall model based on the plurality of dynamic labels and based on a knowledge graph of nutritional products, and constructing a merged recall list that integrates the plurality of recall lists;
   constructing a feature vector used for a sorting model by using the merged recall list and the user portrait, and inputting a calculated feature vector into the sorting model; and
   sorting the merged recall list according to a score calculated by the sorting model for an inputted feature vector, so as to determine the recommendation of personalized nutritional products suitable for the user.

2. The method according to claim 1, wherein the physiological data information in the plurality of dimensions includes one or more of physical examination report data of the user, physiological data of the user received from a wearable device, nutritional requirement data reported by the user himself or herself, and genetic test report data of the user.

3. The method according to claim 2, wherein generating a plurality of static labels that indicate a health state of the user according to the physiological data information in the plurality of dimensions comprises:

   generating a first category label with a first weight according to the physical examination report data of the user, the first category label including a plurality of labels that indicate different physiological health states of the user;
   generating a second category label with a second weight according to the physiological data of the user received from the wearable device, the second category label including a plurality of labels that indicate different physiological activity states of the user;
   generating a third category label with a third weight according to the nutritional requirement data reported by the user himself or herself, the third category label including a plurality of labels that indicate different mental states and nutritional requirements reported by the user himself or herself;
   generating a fourth category label with a fourth weight according to the genetic test report data of the user, the fourth category label including a plurality of labels that indicate different long-term nutritional requirements of the user; and
   generating the plurality of static labels that indicate the health state of the user according to basic information of the user, the first category label, the second category label, the third category label and the fourth category label, wherein the first weight, the second weight, the third weight and the fourth weight have successively decreasing values.

4. The method according to any one of claims 1 to 3, wherein the plurality of behavior data of the user includes a user browsing record, a user searching record, a user question-and-answer record, and a corresponding time record.

5. The method according to claim 4, wherein generating a plurality of dynamic labels that indicate user preferences according to the plurality of behavior data comprises:

   generating browsing labels, searching labels and question-and-answer labels according to user browsing records, user searching records and user question-and-answer records of a plurality of users, the labels including three types: entity, keyword and classification;
   constructing a plurality of sets with browsing labels, searching labels and question-and-answer labels of the plurality of users in a predetermined time period in units of users;
   calculating a score of each user label in the plurality of sets by using a term frequency-inverse document frequency (TF-IDF) algorithm;
   generating a final score of each user label according to the score of each user label, a time attenuation of interaction generation, a weight of interaction type and a weight of a label type;
   selecting a predetermined number of user labels with top-ranked final scores as the dynamic labels of the user.

6. The method according to claim 5, wherein the recall model comprises:

   a singular value decomposition model (SVD)-based SVD recall submodel, which is used to generate an SVD recall list that indicates products that the user is most likely to buy;
   an update recall submodel, which is used to generate an update recall list that indicates latest updated products;
   a popular recall submodel, which is used to generate a popular recall list of popular products according to a click volume and a time decay of products;
   a graph recall submodel, which is used to query products associated with labels from the knowledge graph based on nutritional products according to the plurality of labels of the user, and generate a graph recall list of personalized products; and
   a merging submodel, which is used to merge the SVD recall list, the update recall list, the popular recall list and the graph recall list according to a predetermined merging rule so as to generate a merged fusion list.

7. The method according to claim 6, wherein constructing a feature vector by using the merged recall list and the user portrait comprises:

   performing feature engineering processing on a respective information field in the user portrait; and
   performing weight assignment processing according to a weight of a respective feature, to generate a feature vector for the sorting model, wherein the respective weight in the static labels is greater than the respective weight in the dynamic labels.

8. The method according to claim 7, wherein sorting the merged recall list according to a score calculated by the sorting model for an inputted feature vector, so as to determine the recommendation of personalized nutritional products suitable for the user comprises:

   calculating, by the sorting model, scores that indicate a degree of the user's interest in products according to the inputted feature vector;
   sorting the plurality of recall lists contained in the merged recall list according to values of the scores; and
   determining the nutritional products in the recall list with the highest score as the recommendation of personalized nutritional products suitable for the user.

9. The method according to claim 1, wherein the personalized nutritional products comprise a plurality of combined product units of different types and different quantities of nutritional products corresponding to the health state of the user.

10. The method according to claim 1, further comprising:

determining the health state of the user based on the static labels of the user;

obtaining associated nutritional products based on the health state of the user and the knowledge graph of nutritional products;

determining the recommendation of associated nutritional products suitable for the user, based on the obtained associated nutritional products.

11. The method according to claim 10, further comprising:

displaying the recommendation of personalized nutritional products and/or the recommendation of associated nutritional products to the user, or

transmitting the recommendation of personalized nutritional products and/or the recommendation of associated nutritional products to the user to another electronic device, and

displaying the recommendation of personalized nutritional products and/or the recommendation of associated nutritional products to the user on the another electronic device.

12. The method of claim 1, further comprising:

determining the health state of the user based on the static labels of the user;

displaying a determined health state and a health suggestion corresponding to the health state to the user, or

transmitting the determined health state and a health suggestion corresponding to the health state to the user to another electronic device, and

displaying the determined health state and a health suggestion corresponding to the health state to the user on the another electronic device.

13. The method according to claim 1, further comprising:

determining nutritional requirements of the user based on the static labels of the user;

obtaining associated nutritional products based on the nutritional requirements of the user and the knowledge graph of nutritional products;

determining the recommendation of associated nutritional products suitable for the user based on the obtained associated nutritional products.

14. The method according to claim 13, further comprising:

displaying the nutritional requirements of the user and/or the recommendation of personalized nutritional products to the user, or

transmitting the nutritional requirements of the user and/or the recommendation of personalized nutritional products to the user to another electronic device, and

displaying the nutritional requirements of the user and/or the recommendation of personalized nutritional products to the user on the another electronic device.

15. An apparatus for providing a recommendation of personalized nutrition products, comprising:

a memory on which a computer program is stored; and

a processor configured to, when executing the computer program, perform:

acquiring physiological data information in a plurality of dimensions of a user;

generating a plurality of static labels that indicate a health state of the user according to the physiological data information in the plurality of dimensions;

acquiring a plurality of behavior data of the user;

generating a plurality of dynamic labels that indicate user preferences according to the plurality of behavior data;

generating a user portrait based on the static labels and the dynamic labels;

generating a plurality of recall lists that indicate correspondence between the user and different nutritional products through a trained recall model based on the plurality of dynamic labels and based on a knowledge graph of nutritional products, and constructing a merged recall list that integrates the plurality of recall lists;

constructing a feature vector used for a sorting model by using the merged recall list and the user portrait, and inputting a calculated feature vector into the sorting model; and

sorting the merged recall list according to a score calculated by the sorting model for an inputted feature vector, so as to determine the recommendation of personalized nutritional products suitable for the user.

16. A nonvolatile storage medium having stored thereon a computer program which, when executed by a computer, performs the method according to any one of claims 1-14.

FIG. 1

200

Acquiring physiological data information in a plurality of dimensions of a user
201

Generating a plurality of static labels that indicate a health state of the user according to the physiological data information in the plurality of dimensions
202

Acquiring a plurality of behavior data of the user
203

Generating a plurality of dynamic labels that indicate user preferences according to the plurality of behavior data
204

Generating a user portrait based on the static labels and the dynamic labels
205

Generating a plurality of recall lists that indicate correspondence between the user and different nutritional products through a trained recall model based on the plurality of dynamic labels and based on a knowledge graph of nutritional products, and constructing a recall fusion list that integrates the plurality of recall lists
206

Constructing a feature vector used for a sorting model by using the recall fusion list and the user portrait, and inputting a calculated feature vector into the sorting model
207

Sorting the plurality of recall lists according to a score calculated by the sorting model for an inputted feature vector, so as to determine the recommendation of personalized nutritional products suitable for the user
208

FIG. 2

Article A Browsing Label 1, Label 2, Label 4
Article B Like Label 3, Label 7, Label 8
Article C Favorite Label 2, Label 5, Label 8
Article D Comment Label 3, Label 9

Behavior weight:
Browsing 1
Like 1.5
Favorite 2
Comment 1

Type weight:
Keyword 1
Entity 1.5
Classification 2

Score of label 1=0.7(tfidf score)* time attenuation of interaction generation of Article A
* 1 (behavior weight)*1(type weight)

Score of label 2=0.8(tfidf score)* time attenuation of interaction generation of Article A
* 1 (behavior weight)*1(type weight)+ 0.8(tfidf score)* time attenuation of interaction
generation of Article C* 2 (behavior weight)*2(type weight)

Portrait label
of user A

## FIG. 3

## FIG. 4

FIG. 5

FIG. 6

Computer-executable instructions

**1001**

Computer-readable storage medium **1000**

FIG. 7

Europäisches
Patentamt

European
Patent Office

Office européen
des brevets

# EUROPEAN SEARCH REPORT

**Application Number**

EP 23 17 5844

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/093234 A1 (SHIMA JAMES [US] ET AL) 24 March 2022 (2022-03-24) * paragraphs [0202], [0458], [0200], [0078], [0122], [0341] * | 1-16 | INV. G16H20/60 |
| A | ES 2 644 345 T3 (HOFFMANN LA ROCHE [CH]) 28 November 2017 (2017-11-28) * paragraphs [0049], [0062], [0223], [0090], [0118] * | 1-16 | |
| A | US 2022/378659 A1 (VLEUGELS KATELIJN [US]) 1 December 2022 (2022-12-01) * paragraphs [0084], [0107]; figure 2a * | 1-16 | |
| A | US 2022/253418 A1 (YUAN ZAOSHI AMY [US] ET AL) 11 August 2022 (2022-08-11) * the whole document * | 1-16 | |

**TECHNICAL FIELDS SEARCHED (IPC)**

G16H

The present search report has been drawn up for all claims

| Place of search | Date of completion of the search | Examiner |
|---|---|---|
| Munich | 15 November 2023 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 17 5844**

This annex lists the patent family members relating to the patent documents cited in the above-mentioned European search report.
The members are as contained in the European Patent Office EDP file on
The European Patent Office is in no way liable for these particulars which are merely given for the purpose of information.

**15-11-2023**

| Patent document cited in search report | | | Publication date | Patent family member(s) | | | Publication date |
|---|---|---|---|---|---|---|---|
| US 2022093234 | A1 | | 24-03-2022 | US | 2022093234 | A1 | 24-03-2022 |
| | | | | WO | 2022061145 | A1 | 24-03-2022 |
| ES 2644345 | T3 | | 28-11-2017 | CA | 2687587 | A1 | 31-12-2008 |
| | | | | CN | 101730501 | A | 09-06-2010 |
| | | | | DK | 2170158 | T3 | 18-09-2017 |
| | | | | EP | 2170158 | A2 | 07-04-2010 |
| | | | | ES | 2644345 | T3 | 28-11-2017 |
| | | | | JP | 6017758 | B2 | 02-11-2016 |
| | | | | JP | 2010534494 | A | 11-11-2010 |
| | | | | KR | 20100017924 | A | 16-02-2010 |
| | | | | US | 2009006133 | A1 | 01-01-2009 |
| | | | | WO | 2009002622 | A2 | 31-12-2008 |
| US 2022378659 | A1 | | 01-12-2022 | US | 2020289373 | A1 | 17-09-2020 |
| | | | | US | 2022378659 | A1 | 01-12-2022 |
| US 2022253418 | A1 | | 11-08-2022 | NONE | | | |

EPO FORM P0459

For more details about this annex : see Official Journal of the European Patent Office, No. 12/82